# EUROPÄISCHE PATENTSCHRIFT

(11) **EP 1 534 150 B1**
(45) Veröffentlichungstag und Bekanntmachung des Hinweises auf die Patenterteilung: **01.02.2006**
(21) Anmeldenummer: 03793779.4
(22) Anmeldetag: 30.08.2003
(51) Int. Cl.: A61B 17/16, A61B 17/14

(54) **CHIRURGISCHES INSTRUMENT**
SURGICAL INSTRUMENT
INSTRUMENT CHIRURGICAL

(30) Priorität: 04.09.2002 DE 10240655
(43) Veröffentlichungstag der Anmeldung: 01.06.2005
(73) Patentinhaber: Aesculap AG & Co. KG, 78532 Tuttlingen (DE)
(72) Erfinder: HERMANN, Reiner, 78567 Fridingen (DE)
(74) Vertreter: Boehme, Ulrich
(86) Internationale Anmeldenummer: PCT/EP2003/009664
(87) Internationale Veröffentlichungsnummer: WO 2004/021896

(56) Entgegenhaltungen:
- EP-A- 0 829 237
- FR-A- 1 441 557
- US-A- 3 472 081
- PATENT ABSTRACTS OF JAPAN vol. 2002, no. 08, 5. August 2002 (2002-08-05) & JP 2002 102235 A (BRISTOL-MYERS SQUIBB KK), 9. April 2002 (2002-04-09)

## Beschreibung

Die Erfindung betrifft ein chirurgisches Instrument mit einem oszillierend um eine Drehachse verschwenkbaren Werkzeug und mit einer rotierend angetriebenen Antriebswelle, die über einen Exzenter einen drehfest mit dem Werkzeug verbundenen Schwinghebel oszillierend hin- und herschwenkt.

Derartige chirurgische Instrumente werden beispielsweise für Sägeblätter, Trephine, oszillierende Bohrer oder ähnliche Werkzeuge benutzt, dabei hängt der Winkel der oszillierenden Drehbewegung der Werkzeuge von der Natur der Werkzeuge ab, häufig werden bei Instrumenten mit kleinem Radius relativ große Schwenkwinkel benötigt, bei Werkzeugen mit großem Radius, beispielsweise bei Trephinen, dagegen relativ kleine Schwenkwinkel.

Bei herkömmlichen Instrumenten werden diese unterschiedlichen Schwenkwinkel dadurch erreicht, daß unterschiedliche Handstücke oder Antriebsteile verwendet werden, die jeweils einen bestimmten Schwenkwinkel zur Verfügung stellen. Dokument EP-A-0, 829, 237 (Basis für den Oberbegriff des Anspruchs 1) offenbart einen solchen Antriebsteil. Es gibt auch Handstücke, die eine Verstellung des Schwenkwinkels ermöglichen, dazu ist aber eine komplizierte Verstellmechanik notwendig, die einen sehr aufwendigen Aufbau voraussetzt.

In allen Fällen besteht die Gefahr, daß der Benutzer durch Auswahl eines falschen Handstückes oder durch falsche Verstellung einen Schwenkwinkel auswählt, der für das jeweilige Werkzeug nicht geeignet ist.

Es ist Aufgabe der Erfindung, ein gattungsgemäßes Instrument so auszubilden, daß mit einfachen Mitteln sichergestellt wird, daß ein Werkzeug mit dem für dieses Werkzeug notwendigen Schwenkwinkel betrieben werden kann.

Diese Aufgabe wird bei einem chirurgischen Instrument der eingangs beschriebenen Art erfindungsgemäß dadurch gelöst, daß die Antriebswelle nebeneinander mindestens zwei Exzenter mit verschiedener Exzentrizität aufweist, daß jedem Exzenter ein um die Drehachse des Werkzeuges verschwenkbarer Schwinghebel zugeordnet ist und daß das Werkzeug nur mit einem der Schwinghebel drehfest verbunden ist.

Das chirurgische Instrument stellt also mindestens zwei Schwinghebel zur Verfügung, die von der Antriebswelle mit unterschiedlichem Schwenkwinkel oszillierend verschwenkt werden, und das Werkzeug wird durch geeignete Mittel jeweils nur mit einem dieser Schwinghebel drehfest verbunden, so daß der für dieses Werkzeug erzielbare Schwenkwinkel dem des entsprechenden Schwinghebels entspricht.

Auf diese Weise ist es beispielsweise möglich, bei der Herstellung eines solchen chirurgischen Instrumentes ein universelles Instrument zu verwenden, in das wahlweise unterschiedliche Werkzeuge eingesetzt werden, der für dieses Werkzeug notwendige Schwenkwinkel wird dann vom Hersteller direkt durch drehfeste Verbindung des eingesetzten Werkzeuges mit nur einem der beiden Schwinghebel festgelegt.

Besonders vorteilhaft ist bei dieser Ausgestaltung jedoch eine Möglichkeit, bei der vorgesehen ist, daß bei einem einzigen chirurgischen Instrument unterschiedliche Werkzeuge mit verschiedenen Schwinghebeln drehfest verbunden sind. Es gehören also zu dem chirurgischen Instrument verschiedene Werkzeuge, die mit unterschiedlichen Schwenkwinkeln betrieben werden müssen, und diese sind so ausgebildet, daß sie eine drehfeste Verbindung nur mit dem Schwinghebel im Instrument eingehen, der den für dieses Werkzeug zutreffenden Schwenkwinkel bereitstellt. Auf diese Weise ist es möglich, mit einer einzigen Antriebswelle ohne Verstellung in einfachster Weise unterschiedliche Schwenkwinkel für die verschiedenen Werkzeuge zur Verfügung zu stellen.

Besonders vorteilhaft ist es, wenn das Werkzeug in einer Arbeitslage lösbar mit einem Schwinghebel verbunden ist und aus dieser Arbeitslage aus dem Instrument herausziehbar ist. Damit ist ein einfacher Wechsel des Werkzeuges möglich, beim Einschieben des Werkzeuges verbindet sich dieses zwangsläufig mit dem Schwinghebel, der den für dieses Werkzeug richtigen Schwenkwinkel zur Verfügung stellt, Fehlbedienungen sind nicht möglich, und mit einer Antriebswelle können Werkzeuge unterschiedlicher Schwenkwinkel betrieben werden.

Dabei ist es vorteilhaft, wenn das Werkzeug im Instrument in der eingeschobenen Arbeitslage gegen eine Axialverschiebung fixierbar ist, so daß dadurch die drehfeste Verbindung mit einem bestimmten Schwinghebel sichergestellt ist.

Bei einer besonders bevorzugten Ausführungsform ist vorgesehen, daß die drehfeste Verbindung durch ineinandergreifende radiale Rücksprünge und in diese eintretende, an diesen in Umfangsrichtung anliegende Vorsprünge gebildet wird, die eine axiale Verschiebung des Werkzeugs relativ zur Antriebswelle ermöglicht. Die Lösung und Verbindung der drehfesten Verbindung kann also einfach durch axiale Relativverschiebung der Vorsprünge und der Rücksprünge erfolgen, diese Relativverschiebung ergibt sich durch das Einschieben eines Werkzeuges in das Instrument.

Günstig ist es, wenn der Vorsprung durch eine achsparallele, radial abstehende Rippe gebildet wird.

Der Rücksprung kann vorzugsweise durch eine achsparallele Nut gebildet werden.

Bei einer besonders bevorzugten Ausführungsform ist vorgesehen, daß jeder Schwinghebel einen Rücksprung trägt und daß jedes Werkzeug nur einen Vorsprung aufweist, der in axialer Richtung längs des Werkzeuges so angeordnet ist, daß er eine Drehverbindung nur mit einem Schwinghebel ausbildet. Wenn der Benutzer das Werkzeug in das Instrument einschiebt, steht dieser Vorsprung damit automatisch einer der Nuten der Schwinghebel gegenüber und bildet mit diesem Schwinghebel eine drehfeste Verbindung aus, nicht dagegen mit anderen Schwinghebeln.

Es ist günstig, wenn die Exzenter in unterschiedlicher Winkelstellung angeordnet sind, dadurch kann eine Auswuchtung der Antriebswelle erfolgen und man erhält einen besonders ruhigen Lauf des Instrumentes. Insbesondere ist es günstig, wenn bei zwei Exzentern diese in entgegengesetzter Richtung versetzt sind.

Die nachfolgende Beschreibung bevorzugter Ausführungsformen der Erfindung dient im Zusammenhang mit der Zeichnung der näheren Erläuterung. Es zeigen:
- Figur 1:: eine Längsschnittansicht durch ein chirurgisches Instrument mit zwei nebeneinander angeordneten Schwinghebeln und einem mit dem hinteren Schwinghebel koppelnden Werkzeug;
- Figur 2:: eine Ansicht ähnlich Figur 1 mit einem anderen nur mit dem vorderen Schwinghebel koppelnden Werkzeug und

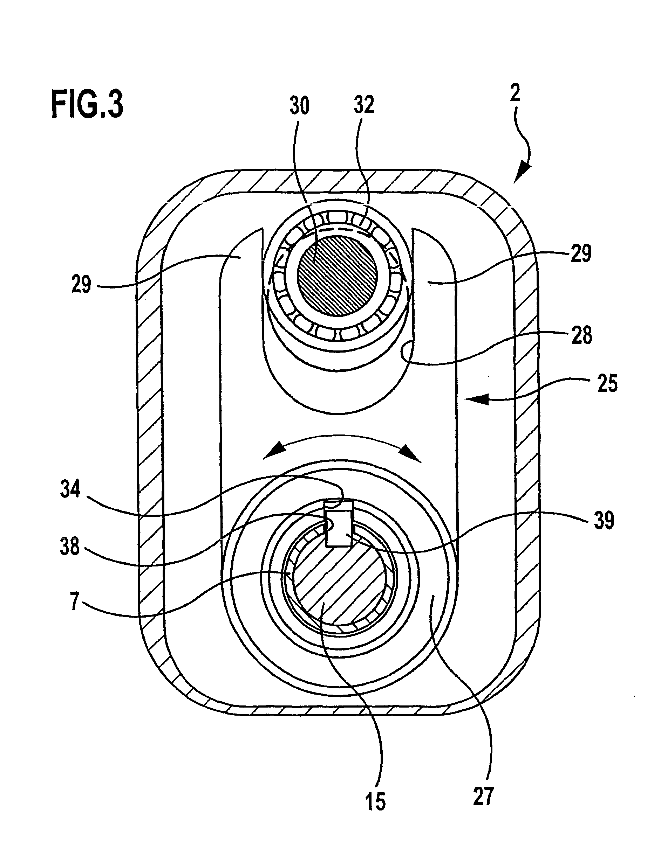

- Figur 3:: eine Schnittansicht längs Linie 3-3 in Figur 2.

Das in der Zeichnung dargestellte chirurgische Instrument 1 umfaßt ein Gehäuse 2, in dem in Kugellagern 3, 4 eine Antriebswelle 5 gelagert ist, die an einem Ende ein Antriebsritzel 6 trägt. Dieses kämmt mit einem Ritzel 7 auf einer Verbindungswelle 8, die ebenfalls über Kugellager 9 im Gehäuse 2 gelagert ist und von einem Anschlußstutzen 10 umgeben wird, über den das Gehäuse 2 mit einer flexiblen Antriebswelle 11 verbunden werden kann. Diese weist einen Mantel 12 und in diesem eine drehbare Welle 13 auf, die von einem in der Zeichnung nicht dargestellten externen Antrieb in Drehung versetzt wird und die drehfest mit der Verbindungswelle 8 gekoppelt werden kann. Auf diese Weise kann die Antriebswelle 5 in kontinuierliche rotierende Bewegung versetzt werden.

Im Gehäuse 2 ist parallel zur Antriebswelle 5 neben dieser eine Aufnahmebohrung 14 für den Schaft 15 eines Werkzeuges 16 angeordnet, diese Aufnahmebohrung 14 ist zu der dem Anschlußstutzen 10 abgewandten Seite des Gehäuses 2 offen, so daß von dieser offenen Seite her der Schaft 15 des Werkzeuges 16 in die Aufnahmebohrung 14 eingeschoben werden kann. In die Aufnahmebohrung 14 ist eine Lagerhülse eingesetzt, die in der Aufnahmebohrung 14 frei verdrehbar ist und die durch einen Querstift 18 in axialer Richtung in der Aufnahmebohrung 14 gesichert ist.

Am offenen Ende der Aufnahmebohrung 14 wird diese von einer Schnellkupplung 19 überfangen, die bei eingeschobenem Werkzeug 16 mit einem Arretierglied 20 in eine Umfangsnut 21 des Werkzeuges 16 eingreift und das Werkzeug dadurch in axialer Richtung in der Lagerhülse 17 und damit in der Aufnahmebohrung 14 festlegt. Die Arretierglieder 20 können in an sich bekannter und hier nicht näher beschriebener Weise dadurch gelöst werden, daß eine Griffhülse 22 gegen die Wirkung einer Feder 23 gedrückt wird, so daß dann das Werkzeug 16 in axialer Richtung aus der Lagerhülse 17 und damit aus der Aufnahmebohrung 14 herausgezogen werden kann.

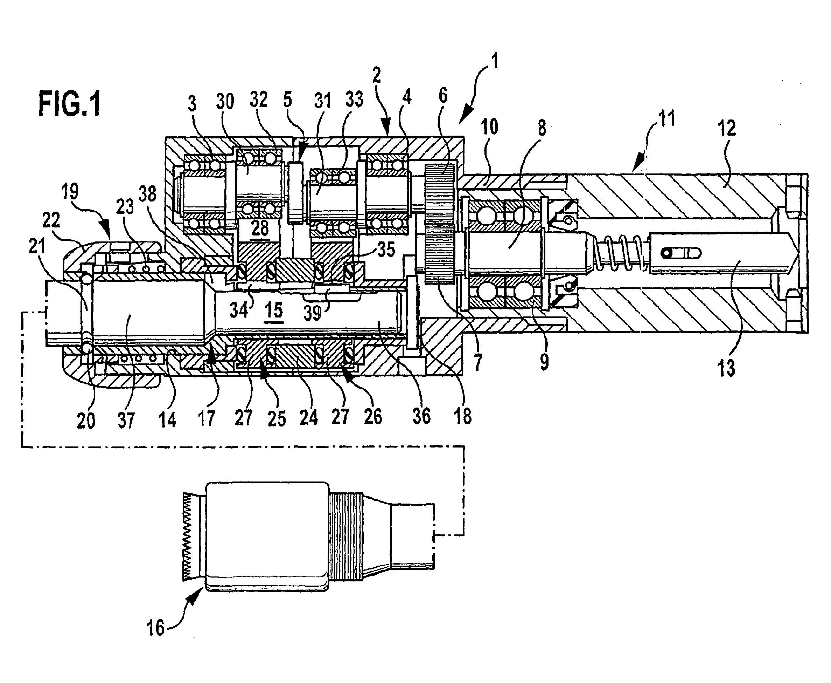

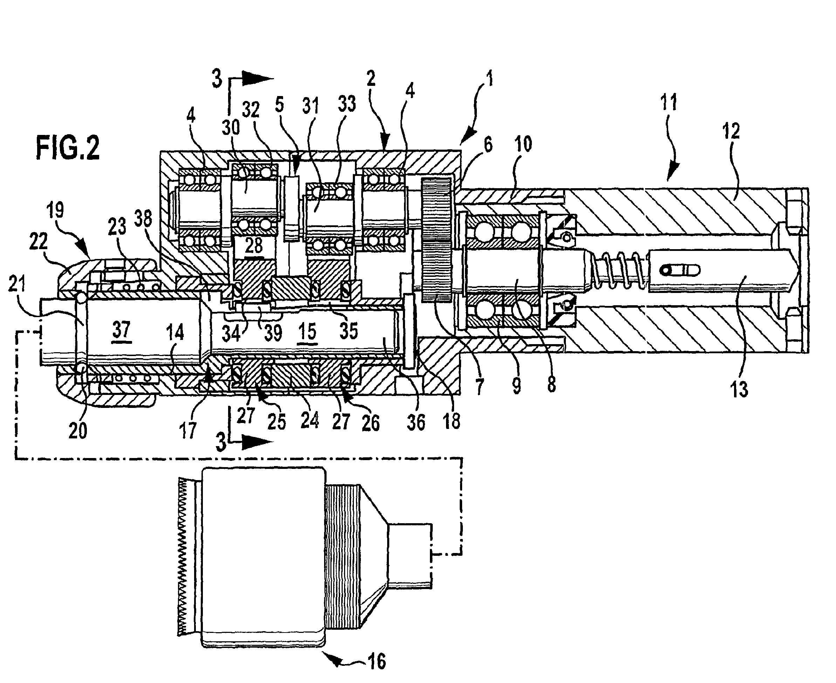

Das Werkzeug 16 kann unterschiedlich ausgebildet sein, im dargestellten Ausführungsbeispiel ist in Figur 1 ein Trephin mit kleinem Durchmesser und in Figur 2 ein Trephin mit einem größeren Durchmesser dargestellt.

Auf der Lagerhülse 17 sind nebeneinander durch einen Distanzring 24 getrennt zwei Schwinghebel 25, 26 verdrehbar gelagert, diese Schwinghebel weisen einen kreisförmigen, die Lagerhülse 17 umgebenden Lagerring 27 auf und zwei parallele, zwischen sich eine einseitig offene Mitnahmeöffnung 28 ausbildende Stege 29 auf, die sich zu beiden Seiten der Antriebswelle 5 erstrecken. Im Bereich der Schwinghebel 25 und 26 weist die Antriebswelle 5 jeweils einen exzentrischen Abschnitt 30 bzw. 31 auf, die Exzentrizität dieser exzentrischen Abschnitte 30 und 31 ist verschieden, die exzentrischen Abschnitte 30 und 31 sind in Umfangsrichtung etwa um 180° gegeneinander versetzt. Jeder exzentrische Abschnitt 30, 31 trägt ein Kugellager 32 bzw. 33, und diese Kugellager 32 und 33 greifen dicht anliegend in die Mitnahmeöffnungen 28 der beiden Schwinghebel 25 bzw. 26 ein, so daß bei einer Verdrehung der Antriebswelle 5 beide Schwinghebel 25, 26 oszillierend hin- und hergeschwenkt werden, und zwar entsprechend der unterschiedlichen Exzentrizität der exzentrischen Abschnitte 30 und 31 mit unterschiedlichem maximalem Schwenkwinkel.

In beiden Schwinghebeln 25, 26 ist an der Innenseite des Lagerrings 27 je eine achsparallele Nut 34, 35 angeordnet, diese beiden Nuten 34 und 35 sind fluchtend miteinander ausgerichtet.

In der Lagerhülse 17 ist in einem hinteren, in die Lagerringe 27 eintauchenden Abschnitt 36, der einen kleineren Durchmesser aufweist als der vordere Abschnitt 37 der Lagerhülse 17, ein achsparalleler Schlitz 38 angeordnet, der sich in Einschubrichtung des Werkzeuges 16 verengt und der sich über die beiden Schwinghebel 25 und 26 hinweg erstreckt.

Das Werkzeug 16 trägt an seinem Schaft 15 einen radial abstehenden Mitnehmer 39, der beim Einschieben des Schaftes 15 in die Lagerhülse 17 in den Schlitz 38 eintritt und in diesem eine gewisse Führung erfährt, der Mitnehmer 39 tritt beim vollständigen Einschieben des Schaftes 15 in die Nut 34 oder die Nut 35 eines der beiden Schwinghebel 25, 26 ein. Bei welcher der beiden Nuten 34, 35 der Mitnehmer 39 eintritt, hängt von der axialen Position des Mitnehmers 39 auf dem Schaft 15 ab. Bei dem Ausführungsbeispiel der Figur 1 ist der Mitnehmer 39 so angeordnet, daß bei vollständig eingeschobenem Schaft 15 der Mitnehmer 39 in die Nut 35 des hinteren Schwinghebels 26 eingreift, bei dem Ausführungsbeispiel der Figur 2 dagegen ist der Mitnehmer 39 mehr zur Vorderseite des Werkzeuges hin angeordnet und tritt dadurch in die Nut 34 des Schwinghebels 25 ein. Der Mitnehmer 39 stellt somit eine drehfeste Verbindung zwischen dem Werkzeug 16 und einem der beiden Schwinghebel 25, 26 her, wobei diese drehfeste Verbindung auch die Lagerhülse 17 erfaßt, da der Mitnehmer 39 durch den Schlitz 38 hindurchtritt. Entsprechend dem jeweiligen Schwenkwinkel des Schwinghebels 25, 26, mit dem diese drehfeste Verbindung erfolgt ist, wird daraufhin das Werkzeug mit unterschiedlichem Schwenkwinkel oszillierend verschwenkt.

Die Auswahl des richtigen Schwinghebels ergibt sich automatisch durch die Positionierung des Mitnehmers am Werkzeug, der Benutzer muß lediglich das jeweilige Werkzeug in das Gehäuse 2 einschieben und mittels der Schnellkupplung 19 in axialer Richtung festlegen, dann ist automatische eine Verbindung mit dem "richtigen" Schwinghebel hergestellt und das Werkzeug wird mit dem Schwenkwinkel angetrieben, der für dieses Werkzeug geeignet ist.

Im dargestellten Ausführungsbeispiel sind lediglich zwei derartige Schwinghebel 25, 26 vorgesehen, grundsätzlich können hier auch mehr Schwinghebel nebeneinander angeordnet sein, denen jeweils ein exzentrischer Abschnitt mit verschiedener Exzentrizität zugeordnet ist und damit ein verschiedener Schwenkwinkel. Der Mitnehmer 39 an den Werkzeugen könnte dann durch unterschiedliche Positionierung selektiv die für dieses Werkzeug zutreffende Verschwenkung auswählen. Damit ergibt sich eine für den Benutzer sehr leicht handhabbare Konstruktion, wenn er ein bestimmtes Werkzeug in das Gehäuse 2 einschiebt, erfolgt automatisch die Drehkopplung mit dem richtigen Schwinghebel, eine Fehlermöglichkeit entfällt, das Werkzeug wählt sich selbst seinen zutreffenden Schwinghebel aus.

## Patentansprüche

1. Chirurgisches Instrument (1) mit einem oszillierend um eine Drehachse verschwenkbaren Werkzeug (16) und mit einer rotierend angetriebenen Antriebswelle (5), die über einen Exzenter (30, 31) einen drehfest mit dem Werkzeug (16) verbundenen Schwinghebel (25, 26) oszillierend hin- und herschwenkt, **dadurch gekennzeichnet, daß** die Antriebswelle (5) nebeneinander mindestens zwei Exzenter (30, 31) mit verschiedener Exzentrizität aufweist, daß jedem Exzenter (30, 31) ein um die Drehachse des Werkzeuges (16) verschwenkbarer Schwinghebel (25, 26) zugeordnet ist und daß das Werkzeug (16) nur mit einem der beiden Schwinghebel (25, 26) drehfest verbunden ist.

2. Instrument nach Anspruch 1, **dadurch gekennzeichnet, daß** unterschiedliche Werkzeuge (16) mit verschiedenen Schwinghebeln (25, 26) drehfest verbindbar sind.

3. Instrument nach Anspruch 1 oder 2, **dadurch gekennzeichnet, daß** das Werkzeug (16) in einer Arbeitslage lösbar mit einem Schwinghebel (25 oder 26) verbunden ist und aus dieser Arbeitslage aus dem Instrument herausziehbar ist.

4. Instrument nach Anspruch 3, **dadurch gekennzeichnet, daß** das Werkzeug (16) im Instrument in der eingeschobenen Arbeitslage gegen eine Axialverschiebung fixierbar ist.

5. Instrument nach einem der voranstehenden Ansprüche, **dadurch gekennzeichnet, daß** die drehfeste Verbindung durch ineinandergreifende radiale Rücksprünge (34, 35) und in diese eintretende, an diesen in Umfangsrichtung anliegende Vorsprünge (39) gebildet wird, die eine axiale Verschiebung des Werkzeuges (16) relativ zur Antriebswelle (5) ermöglicht.

6. Instrument nach Anspruch 5, **dadurch gekennzeichnet, daß** der Vorsprung (39) durch eine achsparallele, radial abstehende Rippe gebildet wird.

7. Instrument nach einem der Ansprüche 5 oder 6, **dadurch gekennzeichnet, daß** der Rücksprung (34, 35) durch eine achsparallele Nut gebildet wird.

8. Instrument nach Anspruch 7, **dadurch gekennzeichnet, daß** jeder Schwinghebel (25, 26) einen Rücksprung (34, 35) trägt und daß jedes Werkzeug (16) nur einen Vorsprung (39) aufweist, der in axialer Richtung längs des Werkzeuges (16) so angeordnet ist, daß er eine Drehverbindung nur mit einem Schwinghebel (25 oder 26) ausbildet.

9. Instrument nach einem der Ansprüche 5 bis 8, **dadurch gekennzeichnet, daß** die Vor- bzw. Rücksprünge (34, 35) an den Schwinghebeln (25, 26) dieselbe Winkelstellung aufweisen.

10. Instrument nach Anspruch 9, **dadurch gekennzeichnet, daß** sich in Einschubrichtung des Werkzeuges (16) verengende Führungsmittel (38) vorgesehen sind, die das Werkzeug (16) so verdrehen, daß die Vorsprünge (39) und Rücksprünge (34, 35) fluchtend ausgerichtet sind.

11. Instrument nach Anspruch 10, **dadurch gekennzeichnet, daß** die Führungsmittel durch eine sich verengende Längsnut (38) in einer das Werkzeug (16) umgebenden Lagerhülse (17) gebildet werden.

12. Instrument nach einem der voranstehenden Ansprüche, **dadurch gekennzeichnet, daß** die Exzenter (30, 31) in unterschiedlicher Winkelstellung angeordnet sind.

13. Instrument nach Anspruch 12, **dadurch gekennzeichnet, daß** bei zwei Exzentern (30, 31) diese in entgegengesetzter Richtung versetzt sind.

## Claims

1. Surgical instrument (1) comprising a tool (16) which is oscillatingly pivotable about an axis of rotation and a rotatingly driven drive shaft (5) which by means of an eccentric (30, 31) causes an oscillating lever (25, 26) which is rotationally fixedly connected to the tool (16) to pivot oscillatingly back and forth, **characterized in that** the drive shaft (5) comprises at least two eccentrics (30, 31) with different eccentricity adjacent to each other, **in that** an oscillating lever (25, 26) pivotable about the axis of rotation of the tool (16) is associated with each eccentric (30, 31), and **in that** the tool (16) is rotationally fixedly connected to only one of the two oscillating levers (25, 26).

2. Instrument in accordance with claim 1, **characterized in that** different tools (16) are rotationally fixedly connectable to different oscillating levers (25, 26).

3. Instrument in accordance with claim 1 or 2, **characterized in that** the tool (16) in a working position is releasably connected to one oscillating lever (25 or 26) and is pullable out of this working position out of the instrument.

4. Instrument in accordance with claim 3, **characterized in that** the tool (16) is securable in the pushed-in working position against axial displacement in the instrument.

5. Instrument in accordance with any one of the preceding claims, **characterized in that** the rotationally fixed connection is formed by radial recesses (34, 35) and projections (39) which enter into and rest against these recesses in circumferential direction interlocking with one another and allowing axial displacement of the tool (16) relative to the drive shaft (5).

6. Instrument in accordance with claim 5, **characterized in that** the projection (39) is formed by an axially parallel, radially protruding rib.

7. Instrument in accordance with claim 5 or 6, **characterized in that** the recess (34, 35) is formed by an axially parallel groove.

8. Instrument in accordance with claim 7, **characterized in that** each oscillating lever (25, 26) carries a recess (34, 35), and **in that** each tool (16) has only one projection (39) which is arranged in axial direction along the tool (16) so as to form a rotational connection with only one oscillating lever (25 or 26).

9. Instrument in accordance with any one of claims 5 to 8, **characterized in that** the angular position of the projections and recesses (34, 35), respectively, on the oscillating levers (25, 26) is the same.

10. Instrument in accordance with claim 9, **characterized in that** guides (38) which narrow in the direction of insertion of the tool (16) are provided to rotate the tool (16) such that the projections (39) and recesses (34, 35) are oriented in alignment with one another.

11. Instrument in accordance with claim 10, **characterized in that** the guides are formed by a narrowing longitudinal groove (38) in a bearing sleeve (17) surrounding the tool (16).

12. Instrument in accordance with any one of the preceding claims, **characterized in that** the eccentrics (30, 31) are arranged in a different angular position.

13. Instrument in accordance with claim 12, **characterized in that** in the case of two eccentrics (30, 31), these are offset in opposite direction.

## Revendications

1. Instrument chirurgical (1) avec un outil (16) pouvant pivoter de manière oscillante autour d'un axe de rotation et avec un arbre d'entraînement (5) entraîné en rotation qui, par l'intermédiaire d'un excentrique (30, 31), fait pivoter de manière oscillante dans un sens et dans l'autre un levier oscillant (25, 26) relié à l'outil (16) de manière solidaire en rotation, **caractérisé en ce que** l'arbre d'entraînement (5) comporte côte à côte au moins deux excentriques (30, 31) d'excentricité différente, qu'à chaque excentrique (30, 31) est associé un levier oscillant (25, 26) pouvant pivoter autour de l'axe de rotation de l'outil (16) et que l'outil (16) n'est relié de manière solidaire en rotation qu'à l'un des deux leviers oscillants (25, 26).

2. Instrument selon la revendication 1 **caractérisé en ce que** différents outils (16) peuvent être reliés de manière solidaire en rotation avec différents leviers oscillants (25, 26).

3. Instrument selon la revendication 1 ou 2 **caractérisé en ce que** l'outil (16) est relié dans une position de travail de manière réversible avec un levier oscillant (25 ou 26) et peut être retiré de l'instrument à partir de cette position de travail.

4. Instrument selon la revendication 3 **caractérisé en ce que** l'outil (16) peut être fixé dans l'instrument dans la position de travail insérée à l'encontre d'un déplacement axial.

5. Instrument selon l'une des revendications précédentes **caractérisé en ce que** la liaison solidaire en rotation est formée par des retraits radiaux coopérants (34, 35) et par des saillies (39) pénétrant dans les précédents et contigus de ceux-ci dans la direction périphérique, qui permet un déplacement axial de l'outil (16) par rapport à l'arbre d'entraînement (5).

6. Instrument selon la revendication 5 **caractérisé en ce que** que la saillie (39) est formée par une nervure parallèle à l'axe en retrait radialement.

7. Instrument selon l'une des revendications 5 ou 6 **caractérisé en ce que** le retrait (34, 35) est formé par une rainure parallèle à l'axe.

8. Instrument selon la revendication 7 **caractérisé en ce que** chaque levier oscillant (25, 26) porte un retrait (34, 35) et **en ce que** chaque outil (16) ne comporte qu'une saillie (39) qui est agencée en direction axiale le long de l'outil (16) de telle manière qu'elle ne forme une liaison à rotation qu'avec un levier oscillant (25 ou 26).

9. Instrument selon l'une des revendications 5 à 8 **caractérisé en ce que** les saillies et les retraits (34, 35) présentent la même position angulaire sur les leviers oscillants (25, 26).

10. Instrument selon la revendication 9 **caractérisé en ce qu'**il est prévu dans la direction d'insertion de l'outil (16) des moyens de guidage (38) qui se rétrécissent, qui soumettent l'outil (16) à une torsion de telle manière que les saillies (39) et les retraits (34, 35) sont orientés de manière alignée.

11. Instrument selon la revendication 10 **caractérisé en ce que** les moyens de guidage sont formés par une rainure longitudinale (38) qui se rétrécit dans une douille de logement (17) entourant l'outil (16).

12. Instrument selon l'une des revendications précédentes **caractérisé en ce que** les excentriques (30, 31) sont disposés dans une position angulaire différente.

13. Instrument selon la revendication 12 **caractérisé en ce que**, dans le cas de deux excentriques (30, 31), ceux-ci sont décalés en direction opposée.
